# EUROPÄISCHE PATENTSCHRIFT

(11) **EP 1 152 762 B1**
(45) Veröffentlichungstag und Bekanntmachung des Hinweises auf die Patenterteilung: **04.05.2005**
(21) Anmeldenummer: 00907513.6
(22) Anmeldetag: 08.02.2000
(51) Int. Cl.: A61K 31/519, A61P 25/28

(54) **VERWENDUNG VON DESOXYPEGANIN ZUR BEHANDLUNG VON ALZHEIMERISCHER DEMENZ**
USE OF DESOXYPEGANINE IN THE TREATMENT OF ALZHEIMER'S DEMENTIA
UTILISATION DE DESOXYPEGANINE DANS LE TRAITEMENT DE LA DEMENCE D'ALZHEIMER

(30) Priorität: 19.02.1999 DE 19906975
(43) Veröffentlichungstag der Anmeldung: 14.11.2001
(73) Patentinhaber: LTS Lohmann Therapie-Systeme AG, 56626 Andernach (DE); HF ARZNEIMITTELFORSCHUNG GmbH, 59368 Werne (DE)
(72) Erfinder: ASMUSSEN, Bodo, D-56170 Bendorf (DE); HILLE, Thomas, D-56567 Neuwied (DE); HOFFMANN, Hans-Rainer, D-56566 Neuwied (DE); OPITZ, Klaus, D-48157 Münster (DE)
(74) Vertreter: Schmidt, Werner, Dr.
(86) Internationale Anmeldenummer: PCT/EP2000/000972
(87) Internationale Veröffentlichungsnummer: WO 2000/048599

(56) Entgegenhaltungen:
- WO-A-97/23484
- DE-A- 19 509 663
- US-A- 5 750 542
- DATABASE WPI Section Ch, Week 197913 Derwent Publications Ltd., London, GB; Class B02, AN 1979-25213B XP002139611 & SU 605 614 A (AS UZB CHEM GROWING), 6. April 1978 (1978-04-06)
- DATABASE WPI Section Ch, Week 198235 Derwent Publications Ltd., London, GB; Class B04, AN 1982-74317E XP002139612 & SU 878 295 A (AS UZB VEGET CHEM), 7. November 1981 (1981-11-07)

## Beschreibung

Die Erfindung ist auf die Verwendung von Desoxypeganin und / oder einem pharmazeutisch annehmbaren Säureadditionssalz von Desoxypeganin zur Herstellung eines Arzneimittels zur Behandlung von Alzheimer'scher Demenz mit gerichtet.

Unter Alzheimer'scher Demenz (Morbus Alzheimer, Senile Demenz vom Alzheimer Typ, SDAT, AD, präsenile Demenz, Alzheimer'sche Krankheit, senile Demenz, primary degenerative dementia, PDD) versteht man die klinische Manifestation einer Störung der am höchsten entwickelten Bereiche des Gehirns. Genauere Beschreibungen sind z. B. bei L. L. Heston et al., Arch. Gen. Psychiatry, (1981) 1085 oder bei R. Terry, R. Katzman: "Senile Dementia of the Alzheimer Type: Defining a Disease" in The Neurology of Aging, ed.: R. Katzman, Chapter 3, p. 51 nachzulesen.

Eine exakte Diagnose von Alzheimer'scher Demenz ist nur postmortal durch Autopsie möglich. Für den Fachmann ist aber aufgrund typischer Symptome (zunehmender Verlust intellektueller Fähigkeiten und Gedächtnisverlust im Frühstadium; Verwirrung, Verlust des Orientierungssinns, abnormale Gefühlslabilität, Depression, Angstzustände, fehlende Motivation, gestörtes Sozialverhalten, Müdigkeit, geschwächte Antriebskraft, Magersucht etc. im fortgeschrittenen Stadium) und bei Abwesenheit von Hinweisen auf andere Ursachen die Alzheimer'sche Demenz mit hoher Wahrscheinlichkeit zu diagnostizieren.
Die Ursache für Alzheimer'sche Demenz ist auch heute noch nicht bekannt. Folglich existiert keine medikamentöse oder sonstige Therapieform für die ursächliche Behandlung der Alzheimer'schen Demenz.

Der von Alzheimer'scher Demenz betroffene Patient, besonders aber auch die unmittelbar mit diesem Patienten zusammenlebenden Angehörigen erleben diese Krankheit als starke Belastung wegen ihres unaufhaltbar fortschreitenden Charakters und weil an ihrem Ende unweigerlich der Tod steht.

Es hat daher viele Strategien gegeben, das Fortschreiten der Alzheimer'schen Demenz zu verlangsamen, d. h. die geistigen (kognitiven) Fähigkeiten zu verbessern. Zu den medikamentösen Strategien zählen die Verabreichung von:
- Psychostimulatien wie Dihydroergotoxin,
- Vasodilatatoren wie Papaverin, Isoxuprin, Cyclandelat,
- Stimulantien wie Methylphenidat, Pentylentetrazol,
- Substanzen zur Verbesserung der cerebralen Durchblutung wie Naftidofuryl, Pentoxifyllin, Suloctidil, Vincamin,
- Calciumkanalblocker wie Nimodipin,
- Nootrope Substanzen wie Piracetam, Oxiracetam, Rolziracetam, Pramiracetam, Aniracetam, CI-844, CI-933,
- Cholinerge Wirkstoffe wie Arecolin, Physostigmin, RS-86, Bethanecol, BM-5,
- Analoge von ACTH wie ORG 2766,
- Vasopressine, wie DDAVP, DGAVP
- Somatostatin, wie L-363,586
- Serotonin-Wirkstoffe wie Alaproclat, Zimelidin,
- adrenerge Substanzen wie Clonidin,
- Hormone wie Oestradiol
aber auch von Vitaminen, Lecithin, Nicotin, Tacrin und anderen.

Aufgabe der vorliegenden Erfindung ist es, ein Arzneimittel zur Verfügung zu stellen, durch die die Symptome von Patienten mit Alzheimer'scher Demenz verringert werden. Insbesondere sollen die kognitiven Fähigkeiten dieser Patienten verbessert bzw. der fortschreitende Charakter der Abnahmne der geistigen Fähigkeiten verlangsamt werden. Die Aufgabe der Erfindung besteht also nicht in einer Behandlung im klassischen Sinne mit der Zielsetzung Heilung, sondern um eine palliative Behandlung der Symptome, um dem Patienten eine weitgehend selbstständige Lebensführung zu ermöglichen und somit den Angehörigen und dem Pflegepersonal die Betreuung des Patienten zu erleichtern.

Gelöst wird die Aufgabe durch die Verwendung, des Wirkstoffs Desoxypeganin und / oder ein pharmazeutisch annehmbares Säureadditionssalz zur Herstellung eines Arzneimitels zur Behandlung von Alzheimer'scher Demenz

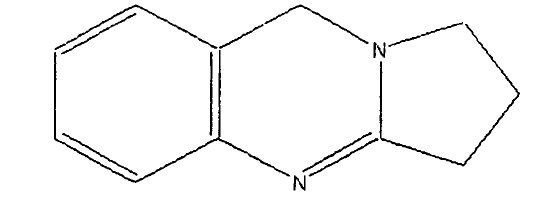

Bei Desoxypeganin handelt es sich um ein Alkaloid der Summenformel C₁₁H₁₂N₂ mit obiger Struktur, das in Pflanzen aus der Familie der Zygophyllaceae enthalten ist. Als Säureadditionsssalz (Hydrochlorid, C₁₁H₁₂N₂ · HCl · H₂O) ist es kommerziell erhältlich.

Desoxypeganin ist in der ehemaligen Sowjetunion zwar ausführlich untersucht und seine pharmakologischen Wirkungen intensiv erforscht worden, die erfindungsgemässe Verwendung einer desoxypeganin-haltigen Formulierung zur Behandlung von Patienten mit Alzheimer'scher Demenz wurde aber bisher nicht beschrieben.

Aufgrund seiner pharmakologischen Eigenschaften gehört Desoxypeganin zur Gruppe der reversibel wirkenden Cholinesterasehemmstoffe, steht in seinen Wirkungen dem Physostigmin, dem Neostigmin und dem Galanthamin nahe, zeichnet sich jedoch durch besondere spezifische Eigenschaften aus. Desoxypeganin hemmt nämlich nicht nur die Acetylcholinesterase und damit den Abbau von Acetylcholin, sondern auch die Monoaminoxydase und damit den Abbau von Dopamin. Dieser Vorteil wiegt seine auf die Gewichtseinheit bezogene etwas geringere Cholinesterasehemmwirkung (im Vergleich zu Physostigmin) auf.

Im Gegensatz zu Neostigmin überwindet Desoxypeganin die Bluthirnschranke und antagonisiert die cerebralen Wirkungen cholinerger Gifte.

Die Gewinnung des Desoxypeganin erfolgt durch Isolierung aus der Steppenraute (Peganum harmala) oder durch Synthese.

in der vorliegenden Erfindung werden Arzneiformen eingesetzt, die den Wirkstoff vorzugsweise kontrolliert (retardiert) freisetzen. Besonders bevorzugt sind Arzneiformen, die den Wirkstoff über einen längeren Zeitraum, z. B. etwa 12, 16, 24, 48 oder 72 Stunden abgeben. Auch implantierte Vorrichtungen, die den Wirkstoff über einen Zeitraum von mehreren Tagen, Wochen oder sogar Monaten abgeben können, sind erfindungsgemäß einsetzbar. Generell sind solche Arzneiformen im Stand der Technik bekannt.

Die Verabreichung pharmazeutisch wirksamer Verbindungen mittels solcher Formulierungen kann oral, transdermal oder anderweitig parenteral (z. B. intracerebroventrikulär, intravenös, rektal), soll aber vorzugsweise protahiert erfolgen.

Das erfindungsgemäße Arzneimittel für die Behandlung von Alzheimer'scher Demenz ist dadurch gekennzeichnet, daß Desoxypeganin und / oder ein pharmazeutische annehmbares Säureadditionssalz in einer wirksamen Menge enthalten ist.

Bei derartigen Arzneimitteln kann das Desoxypeganin als solches oder in Form pharmazeutisch annehmbarer Säureadditonssalze vorliegen z. B. als Hydrohalogenid, insbesondere -chlorid oder -bromid, oder als Salz einer anderen pharmazeutisch annehmbaren Säure, z. B. als Citrat, Tartrat, Acetat.

Diese Arzneiformen enthalten ferner in der Regel Hilfsstoffe, wie Trägerstoffe, Fliessverbesserer, Lösungsmittel und Öle, deren Art und Menge je nach Darreichungsform schwankt.

Im allgemeinen liegt der Gehalt an Wirkstoff im Arzneimittel, berechnet als freies Desoxypeganin, zwischen 0,1 und 50 Gew.-%, vorzugsweise zwischen 2 und 15 Gew.-%.

Die Behandlung von Alzheimer'scher Demenz ist dadurch gekennzeichnet, daß einem Patienten, der an den Symptomen der Alzheimer'schen Demenz leidet, ein Arzneimittel enthaltend Desoxypeganin und / oder ein pharmazeutisch annehmbares Säureadditionssalz von Desoxypeganin verabreicht wird. In einer weiteren Ausführungsform dieser Behandlung wird ein solches Arzneimittel in Kombination mit einem im Stand der Technik bekannten Arzneimittel zur Behandlung der Alzheimer'schen Demenz eingesetzt.

## Patentansprüche

1. Verwendung von Desoxypeganin und / oder einem pharmazeutisch annehmbaren Säureadditionssalz von Desoxypeganin zur Herstellung eines Arzneimittels für die Behandlung von Alzheimer'scher Demenz.

2. Verwendung nach Anspruch 1, **dadurch gekennzeichnet, dass** der Gehalt an Wirkstoff, berechnet als freies Desoxypeganin, in einer Menge zwischen 0,1 und 50 Gew.-%, vorzugsweise zwischen 2 und 15 Gew.-%, im Arzneimittel enthalten ist.

3. Verwendung nach Anspruch 1 oder 2, **dadurch gekennzeichnet, dass** der Wirkstoff in einer wirksamen Menge in Form eines Säureadditionssalzes, insbesondere als Hydrochlorid oder -bromid, vorliegt.

4. Verwendung nach einem der vorangehenden Ansprüchen, **dadurch gekennzeichnet, dass** der Wirkstoff in einer oral, transdermal oder parenteral applizierbaren Form vorliegt.

5. Verwendung nach einem der vorangehenden Ansprüchen, **dadurch gekennzeichnet, dass** die Abgabe des Wirkstoffes protahiert erfolgt.

6. Verwendung nach Anspruch 5, **dadurch gekennzeichnet, dass** die Abgabe des Wirkstoffes über einen Zeitraum von mindestens 12 Stunden erfolgt.

## Claims

1. Use of deoxypeganine and/or a pharmaceutically acceptable acid addition salt of deoxypeganine for producing a medicament for the treatment of Alzheimer's dementia.

2. Use according to Claim 1, **characterized in that** the content of active compound calculated as free deoxypeganine, in the medicament is between 0.1 and 50% by weight and preferably between 2 and 15% by weight.

3. Use according to Claim 1 or 2, **characterized in that** the active compound is present in an efficacious amount in the form of an acid addition salt, in particular as hydrochloride or hydrobromide.

4. Use according to any one of the preceding claims, **characterized in that** the active compound is present in an orally, transdermally or parenterally administrable form.

5. Use according to any one of the preceding claims, **characterized in that** the active compound is administered over a prolonged period.

6. Use according to Claim 5, **characterized in that** the active compound is administered for not less than 12 hours.

## Revendications

1. Utilisation de désoxypéganine et/ou d'un sel d'addition acide de désoxypéganine pharmaceutiquement acceptable pour la fabrication d'un médicament destiné au traitement de la maladie d'Alzheimer.

2. Utilisation selon la revendication 1, **caractérisée en ce que** la quantité de principe actif, par rapport à la désoxypéganine libre, contenue dans le médicament est de 0,1 à 50% en poids, de préférence de 2 à 15% en poids.

3. Utilisation selon la revendication 1 ou 2, **caractérisée en ce que** le principe actif est présent en une quantité efficace sous forme d'un sel d'addition acide, en particulier un chlorhydrate ou un bromhydrate.

4. Utilisation selon l'une des revendications précédentes, **caractérisée en ce que** le principe actif est présent sous une forme d'administration par voie orale, transdermique ou parentérale.

5. Utilisation selon l'une des revendications précédentes, **caractérisée en ce que** la délivrance du principe actif a lieu de façon prolongée.

6. Utilisation selon la revendication 5, **caractérisée en ce que** la délivrance du principe actif a lieu sur une durée d'au moins 12 heures.
